# EUROPÄISCHE PATENTSCHRIFT

(11) **EP 1 891 049 B1**
(45) Veröffentlichungstag und Bekanntmachung des Hinweises auf die Patenterteilung: **19.07.2017**
(21) Anmeldenummer: 06753669.8
(22) Anmeldetag: 17.05.2006
(51) Int. Cl.: C07D 403/04

(54) **VERFAHREN ZUR HERSTELLUNG VON 2-(2-AMINO-PYRIMIDIN-4-YL)-1H-INDOL-5-CARBONSÄURE-DERIVATEN**
METHOD FOR PRODUCING 2-(2-AMINO-PYRIMIDIN-4-YL)-1H-INDOLE-5-CARBOXYLIC ACID DERIVATIVES
PROCEDE DE PRODUCTION DE DERIVES D'ACIDE 2-(2-AMINO-PYRIMIDINE-4-YLE)-1H-INDOL-5-CARBOXYLIQUE

(30) Priorität: 01.06.2005 DE 102005025225
(43) Veröffentlichungstag der Anmeldung: 27.02.2008
(60) Teilanmeldung: 10163868.2
(73) Patentinhaber: SANOFI, 75008 Paris (FR)
(72) Erfinder: GRAESER, Joachim, 65926 Frankfurt am Main (DE); BILLEN, Guenter, 65926 Frankfurt am Main (DE); LINKIES, Adolf, 65926 Frankfurt am Main (DE); METZENTHIN, Tobias, 65926 Frankfurt am Main (DE)
(74) Vertreter: Then, Johann
(86) Internationale Anmeldenummer: PCT/EP2006/004645
(87) Internationale Veröffentlichungsnummer: WO 2006/128585

(56) Entgegenhaltungen:
- EP-A2- 1 127 874
- WO-A-2004/022553
- DATABASE CA [Online] CHEMICAL ABSTRACTS SERVICE, COLUMBUS, OHIO, US; XP002397851 gefunden im STN Database accession no. 2000:499651 in der Anmeldung erwähnt & JIANG, BIAO ET AL: "Synthesis of indolylpyrimidines via cross-coupling of indolylboronic acid with chloropyrimidines: facile synthesis of meridianin D" HETEROCYCLES , CODEN: HTCYAM; ISSN: 0385-5414, Bd. 53, Nr. 7, 2000, Seiten 1489-1498,
- HIRAO K ET AL: "Rhodium-Catalyzed Carbonylation of 2-Alkynylaniline: Syntheses of 1,3-Dihydroindol-2-ones" TETRAHEDRON LETTERS, ELSEVIER, AMSTERDAM, NL, Bd. 36, Nr. 35, 28. August 1995 (1995-08-28), Seiten 6243-6246, XP004027359 ISSN: 0040-4039
- MIRCO COSTA ET AL: "Synthesis of 4 H -3,1-Benzoxazines, Quinazolin-2-ones, and Quinoline-4-ones by Palladium-Catalyzed Oxidative Carbonylation of 2-Ethynylaniline Derivatives", THE JOURNAL OF ORGANIC CHEMISTRY, vol. 69, no. 7, 1 April 2004 (2004-04-01), pages 2469-2477, XP055009153, ISSN: 0022-3263, DOI: 10.1021/jo0357770
- ARCADI A ET AL: "Palladium-catalysed coupling of aryl and viny triflates or halides with 2-ethynylaniline: An efficient route to functionalized 2-substituted indoles", TETRAHEDRON LETTERS, PERGAMON, GB, vol. 30, no. 19, 1 January 1989 (1989-01-01), pages 2581-2584, XP002258672, ISSN: 0040-4039, DOI: 10.1016/S0040-4039(01)80456-1
- KORADIN C ET AL: "Synthesis of polyfunctional indoles and related heterocycles mediated by cesium and potassium bases", TETRAHEDRON, ELSEVIER SCIENCE PUBLISHERS, AMSTERDAM, NL, vol. 59, no. 9, 24 February 2003 (2003-02-24), pages 1571-1587, XP004409958, ISSN: 0040-4020, DOI: 10.1016/S0040-4020(03)00073-5

## Beschreibung

Die Erfindung betrifft neue Zwischenprodukte bei der Gewinnung der Verbindung der Formel I. Die genannten Indolderivate sind geeignete Zwischenprodukte zur Herstellung von IkB-Kinase Inhibitoren (WO 01/30774 A1; WO2004/022553).

Es ist bekannt, dass man Indolderivate als Bausteine für die Synthese von pharmazeutischen Wirkstoffen einsetzt. Beispielsweise sind 2-(2-Amino-pyrimidin-4-yl)-1H-indol-5-carbonsäuren oder deren Salze wichtige Bausteine für die Herstellung von IkB-Kinase Inhibitoren (siehe WO 01/30774 A1):

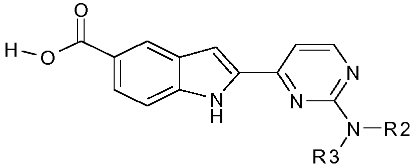

2-(2-Amino-pyrimidin-4-yl)-1H-indol-5-carbonsäuren können durch klassische Fischer-Indolsynthese ausgehend von den entsprechenden 4-Acetylpyrimidinen (III) und 4-Hydrazinobenzoesäure (II) hergestellt werden (siehe Schema 1):

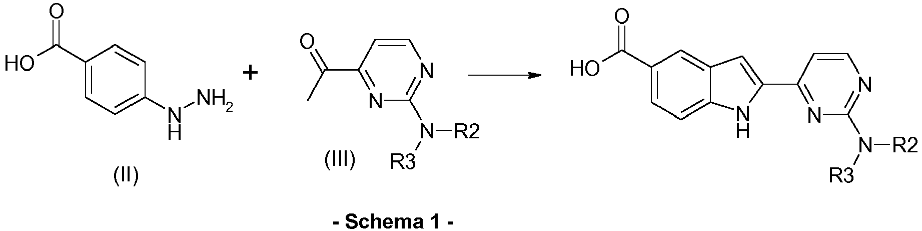

Nachteilig sind hierbei zum einen die drastischen Reaktionsbedingungen, die für eine vollständige Umsetzung benötigt werden. Zum anderen fallen die Produkte dieser Reaktion im Gemisch mit den entsprechenden Oligomeren an, was zu einer schlechten Isolierbarkeit führt, insbesondere in Hinblick auf die Filtrationszeit. Ferner lassen sich diese Oligomere aufgrund der geringen Löslichkeit von 2-(2-Amino-pyrimidin-4-yl)-1H-indol-5-carbonsäuren in organischen Lösungsmitteln nur schlecht abtrennen und werden bei den weiteren Reaktionen teilweise bis zum Wirkstoff als Verunreinigung mitgeschleppt.

In Arcadi A et al., Tetrahedron Letters, 30(19), 1989, 2581-2584; Koradi C. et al., Tetrahedron, 59(9), 2003, 1571-1587 und in EP 1 127 874 wird die Synthese von funktionalisierten Indolen durch Zyklisierung von 2-Alkinylanilinen beschreiben. In Mirco Costa et al, The Journal of Organic Chemistry, 69(7), 2004, 2469-2477 wird 4-Methoxycarbonyl-2-(trimethylsilylethynyl)anilin als Edukt zur die Synthese bestimmter Chinolin-4-on-Derivate offenbart.

Aufgabe der vorliegenden Erfindung ist es, ein technisches Verfahren zur Herstellung der Verbindungen der Formel I zu finden, welche die genannten Nachteile nicht aufweist.

Es wurden die folgenden Verfahren zur Gewinnung der Verbindung der Formel I gefunden, 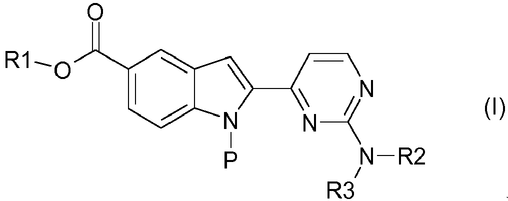 wobei
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
R2 und R3 gleich oder verschieden sind und unabhängig voneinander für
   1) Wasserstoffatom,
   2) -(C₁-C₁₂)-Alkyl,
   3) -(C₆-C₁₄)-Aryl, wobei Aryl unsubstituiert oder ein, zwei oder dreifach durch -(C₁-C₆)-Alkyl substituiert ist,
   4) -(C₃-C₈)-Cycloalkyl oder
   5) 4- bis 15-gliedriger Het-Ring, stehen,
P für Wasserstoffatom oder für eine N-Schutzgruppe steht,
   das dadurch gekennzeichnet ist, dass man
   a) ein Boronoindol der Formel IV 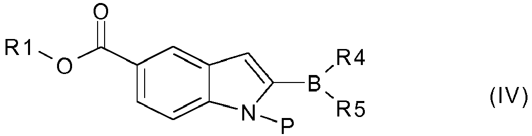
      worin R1 die gleiche Bedeutung wie in Formel I hat,
      R4 und R5 gleich oder verschieden sind und unabhängig voneinander für
         1) -OH,
         2) -O-(C₁-C₁₂)-Alkyl,
         3) -O-(C₆-C₁₄)-Aryl,
         4) -O-(C₃-C₈)-Cycloalkyl,
         5) -(C₁-C₁₂)-Alkyl oder
         6) -O-Het, wobei Het ein 4- bis 15-gliedriger Het-Ring bedeutet, stehen, oder R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
      P für Wasserstoffatom oder für eine N-Schutzgruppe steht,
         mit einem Aminopyrimidin der Formel V umsetzt, 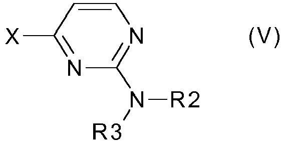
      worin R2 und R3 die gleiche Bedeutung wie in Formel I haben und X für
         1) Halogen,
         2) -O-SO₂-R2, oder
         3) -O-C(O)-R2 steht,
      und die gegebenenfalls vorhandene N-Schutzgruppe abspaltet, oder
   b) ein Boronoindol der Formel IV mit einem Pyrimidin der Formel VI, 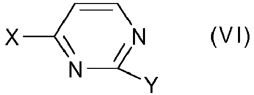
      worin X die gleiche Bedeutung wie in Formel V hat und Y für
         1) Halogen,
         2) -O-SO₂-R2, oder
         3) -O-C(O)-R2 steht,
      zu einer Verbindung der Formel VII umsetzt, 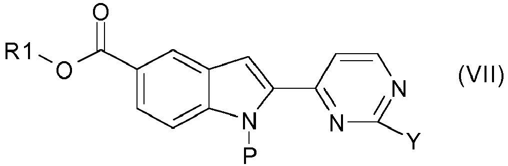
      worin R1 und P die Bedeutung in der Verbindung der Formel IV haben und Y die gleiche Bedeutung wie in der Verbindung der Formel VI hat,
      und anschließend die Verbindung der Formel VII mit einem Amin der Formel VIII 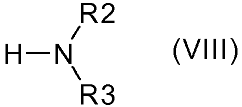
      worin R2 und R3 die gleiche Bedeutung wie in Formel I haben,
      zu einer Verbindung der Formel I umsetzt und die gegebenenfalls vorhandene N-Schutzgruppe abspaltet, oder
   c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

Das Verfahren a) ist zur Gewinnung der Verbindung der Formel I geeignet, wobei
R1 für
1) Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für Wasserstoffatom oder
- (C₁-C₄)-Alkyl, stehen,
das dadurch gekennzeichnet ist, dass man ein Boronoindol der Formel IV,
worin R1 wie für Formel I definiert ist,
R4 und R5 gleich oder verschieden sind und unabhängig voneinander für -OH oder
- (C₁-C₆)-Alkyl, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für
1) Wasserstoffatom,
2) -C(O)-O-R6, worin R6 für
   a) Wasserstoffatom,
   b) -(C₁-C₆)-Alkyl,
   c) -(C₆-C₁₄)-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
   d) -(C₃-C₆)-Cycloalkyl oder
   e) für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
3) -SO₃-R6,
4) -O-SO₂-R6,
5) -Si-R6 oder
6) Benzyl steht,
mit einem Aminopyrimidin der Formel V umsetzt,
worin R2 und R3 gleich oder verschieden sind und unabhängig voneinander für Wasserstoffatom oder -(C₁-C₄)-Alkyl stehen, und
X für Cl, Br, I, -O-Tosylat, -O-Mesylat oder -O-Acetat steht, und
die gegebenenfalls vorhandene N-Schutzgruppe abspaltet.

Das Verfahren ist weiterhin zur Gewinnung der Verbindung der Formel I geeignet, wobei R1 für Wasserstoffatom oder Ethyl steht,
R2 und R3 gleich oder verschieden sind und unabhängig voneinander für Wasserstoffatom oder
- (C₁-C₄)-Alkyl, stehen,
das dadurch gekennzeichnet ist, dass man ein Boronoindol der Formel IV,
worin R1 wie für Formel I definiert ist,
R4 und R5 gleich sind und für -OH stehen, und
P für
1) Wasserstoffatom,
2) -O-Tosylat oder
3) Benzyl steht,
mit einem Aminopyrimidin der Formel V umsetzt,
worin R2 und R3 gleich oder verschieden sind und unabhängig voneinander für Wasserstoffatom oder -(C₁-C₄)-Alkyl stehen, und
X für Cl, Br, I, -O-Tosylat, -O-Mesylat oder -O-Acetat steht und
die gegebenenfalls vorhandene N-Schutzgruppe abspaltet.

Das Verfahren b) ist zur Gewinnung der Verbindung der Formel I geeignet, wobei R1 für
1) Wasserstoffatom,
2) -(C₁-C₆)-Alkyl,
3) Phenyl oder
4) -(C₃-C₆)-Cycloalkyl, steht,
R2 und R3 gleich oder verschieden sind und unabhängig voneinander für Wasserstoffatom oder
- (C₁-C₄)-Alkyl, stehen,
das dadurch gekennzeichnet ist, dass man ein Boronoindol der Formel IV,
worin R1 wie für Formel I definiert ist,
R4 und R5 gleich oder verschieden sind und unabhängig voneinander für -OH oder
- (C₁-C₆)-Alkyl, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan,
P für
1) Wasserstoffatom,
2) -C(O)-O-R6, worin R6 für
   a) Wasserstoffatom,
   b) -(C₁-C₆)-Alkyl,
   c) -(C₆-C₁₄₎-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
   d) -(C₃-C₆)-Cycloalkyl oder
   e) für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
3) -SO₃-R6,
4) -O-SO₂-R6,
5) -Si-R6 oder
6) Benzyl steht,
mit einem Pyrimidin der Formel VI, worin
X für Cl, Br, I, -O-Tosylat, -O-Mesylat oder -O-Acetat steht, und
Y für Cl, Br, I, -O-Tosylat, -O-Mesylat oder -O-Acetat steht,
zu einer Verbindung der Formel VII umsetzt und anschließend die Verbindung der Formel VII mit einem Amin der Formel VIII, worin R2 und R3 gleich oder verschieden sind und unabhängig voneinander für Wasserstoffatom oder -(C₁-C₄)-Alkyl stehen, zu einer Verbindung der Formel I umsetzt und die gegebenenfalls vorhandene N-Schutzgruppe abspaltet.

Das Verfahren ist ferner zur Gewinnung der Verbindung der Formel I geeignet, wobei R1 für Wasserstoffatom oder Ethyl steht,
R2 und R3 gleich oder verschieden sind und unabhängig voneinander für Wasserstoffatom oder
- (C₁-C₄)-Alkyl, stehen,
das dadurch gekennzeichnet ist, dass man ein Boronoindol der Formel IV,
worin R1 wie für Formel I definiert ist,
R4 und R5 gleich sind und für -OH stehen,
P für
1) Wasserstoffatom,
2) -O-Tosylat oder
3) Benzyl steht,
mit einem Pyrimidin der Formel VI, worin
X für Cl, Br, I, -O-Tosylat, -O-Mesylat oder -O-Acetat steht, und
Y für Cl, Br, I, -O-Tosylat, -O-Mesylat oder -O-Acetat steht, zu einer Verbindung der Formel VII umsetzt und anschließend die Verbindung der Formel VII mit einem Amin der Formel VIII, worin R2 und R3 gleich oder verschieden sind und unabhängig voneinander für Wasserstoffatom oder -(C₁-C₄)-Alkyl stehen, zu einer Verbindung der Formel I umsetzt und die gegebenenfalls vorhandene N-Schutzgruppe abspaltet.

Der Verfahrensschritt a) wird beispielsweise gemäß den Reaktionsbedingungen wie sie von B. Jiang und C. Yang in Heterocycles, Vol. 53, 2000, S. 1489-1498 beschrieben sind, durchgeführt. Die Umsetzung der Boronoindole (IV) mit den Pyrimidinderivaten (V) wird bevorzugt in Gegenwart katalytischer Mengen an Palladium- oder Nickelverbindungen wie Pd(PPh₃)₄, Pd₂(dba)₃, Pd(OAc)₂ oder PdCl₂/TPPTS durchgeführt.

Die Reaktionstemperatur beträgt dabei von 40 °C bis 80 °C, bevorzugt von 60 °C bis 70 °C. Die Reaktionszeit liegt im allgemeinen von 2 bis 3 Stunden, je nach Zusammensetzung des Gemisches und des gewählten Temperaturbereiches. Als Lösungsmittel eignen sich beispielsweise Methanol, Ethanol oder Toluol. Das molare Verhältnis der Verbindung der Formel IV zur Verbindung der Formel V liegt beispielsweise bei 1:1 bis 1:1,3.
Die Reinheit wird durch HPLC ermittelt.

Die Verbindungen der Formel IV sind entweder bekannt oder lassen sich beispielsweise durch Umsetzung der entsprechenden N-geschützten Indol-5-carbonsäurederivate (XI) mit Basen wie LDA, LiTMP oder LiHMDS und anschließende Umsetzung mit Borsäureestern wie Triisopropylborat oder Trimethylborat herstellen (Schema 5).

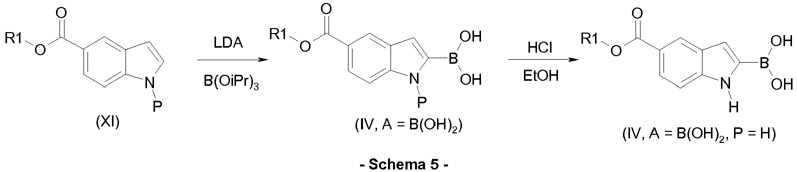

Es hat sich als vorteilhafter erwiesen, vor der eigentlichen Umsetzung die N-Schutzgruppe zu entfernen, da in diesem Fall das während der Kupplung durch Abspaltung der Borono-Gruppe als Nebenprodukt entstehende Indol (XI) in geringerem Maße gebildet wird, was in einer höheren Ausbeute resultiert.

Die Verbindungen der Formel V sind entweder bekannt oder lassen sich durch Umsetzung von Pyrimidinderivaten (VI) mit Aminen VIII und anschließende Abtrennung vom Isomeren XII, beispielsweise durch Chromatographie oder Wasserdampfdestillation, herstellen (siehe Schema 6)

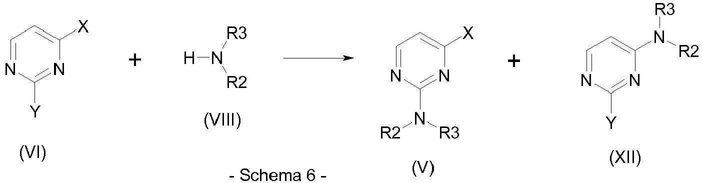

Es ist auch möglich, die eigentliche Umsetzung mit Gemischen von (V) und (XII) durchzuführen, da sich überraschenderweise gezeigt hat, dass das Isomere (XII) unter den Reaktionsbedingungen dieser Kupplung nicht zu dem zu (I) isomeren (XIII) reagiert (Schema 7) 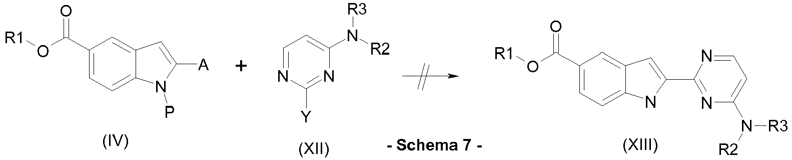 Der Verfahrensschritt b) wird analog zu a) durchgeführt

Die Herstellung physiologisch verträglicher Salze aus zur Salzbildung befähigten Verbindungen der Formel I, einschließlich deren stereoisomeren Formen, erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin
oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin-, oder Trifluoressigsäure in Frage.
Bevorzugt sind Alkali- und Erdalkalimetalle oder Ammoniumsalze. Beispiele für Alkali- und Erdalkalimetalle sind Li, Na, K, Rb, Cs, Be, Mg, Ca, Sr oder Ba. Beispiel für Ammoniumsalz ist NH₄.

Unter dem Begriff "N-Schutzgruppe" für P werden übliche Aminschutzgruppen, wie sie in T. Greene, "Protective Groups in Organic Synthesis" beschrieben sind, verstanden. Weitere Beispiele für N-Schutzgruppen sind die Reste 2) bis 6) für P als Rest in der Verbindung der Formel II.
Unter dem Begriff "katalytischer Mengen an Palladium- oder Nickelverbindungen" werden von 0,03 bis 0,3 Mol (z.B. bezogen auf Mol der Verbindung der Formel IV) der folgenden Verbindungen Pd(PPh₃)₄, Pd₂(dba)₃, Pd(OAc)₂ oder PdCl₂/TPPTS verstanden.
Unter dem Begriff "Metallkatalysatoren" werden zum Beispiel von 0,03 bis 0,3 Mol (beispielsweise bezogen auf Mol der Verbindung der Formel IV) der folgender Verbindungen Pd(PPh₃)₄, Pd₂(dba)₃, Pd(OAc)₂ oder PdCl₂/TPPTS verstanden.
Unter den Begriffen "-(C₁-C₁₂)-Alkyl" werden Kohlenwasserstoffreste verstanden, deren Kohlenstoffkette geradkettig oder verzweigt ist und 1 bis 12 Kohlenstoffatome enthält. Beispiele sind Methyl, Ethyl, Propyl, Iso-Propyl, Butyl, Iso-Butyl, tertiär-Butyl, Pentyl, Neopentyl, Hexyl, 2,3-Dimethylbutan, Neohexyl, Heptyl, Octyl, Nonanyl, Decanyl oder Dodecanyl.
Unter dem Begriff "(C₃-C₈)-Cycloalkyl" werden Reste verstanden wie Verbindungen, die sich von 3- bis 8-gliedrige Monocyclen wie Cyclopropyl, Cyclobutyl, Cyclopentyl, Cyclohexyl, Cycloheptyl oder Cyclooctyl herleiten.
Unter den Begriffen "-(C₆-C₁₄)-Aryl," oder "Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.

Unter dem Begriff "4- bis 15-gliedriger Het-Ring" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydro-chinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzo-furanyl, Isochromanyl, Isoindazolyl, Isoindolinyl, Isoindolyl, Isochinolinyl, Benzimidazolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl.
Unter dem Begriff "R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatomen zwei Sauerstoffatome oder zwei Sauerstoffatome und ein Stickstoffatom enthalten," werden Ringsysteme verstanden, die sich beispielsweise von Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan ableiten.

Ferner treten bei dem beschriebenen Verfahren neue Verbindungen der Formel II, 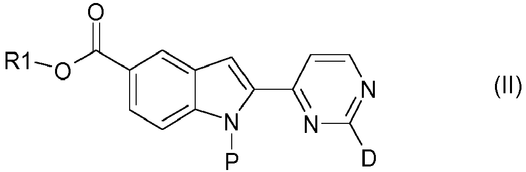 oder ein physiologisch verträgliches Salz der Verbindung der Formel II auf, wobei
R1 für
1) Wasserstoffatom,
2) -(C₁-C₁₂)-Alkyl,
3) -(C₆-C₁₄)-Aryl,
4) -(C₃-C₈)-Cycloalkyl oder
5) 4- bis 15-gliedriger Het-Ring, steht,
mit der Maßgabe, dass R1 nicht Wasserstoffatom ist, wenn P für Wasserstoffatom steht und D für -N(R2)-R3 steht, worin R2 für Wasserstoffatom steht und R3 für -(C₁-C₄)-Alkyl steht, P für
1) Wasserstoffatom,
2) -C(O)-O-R6, worin R6 für
   a) Wasserstoffatom,
   b) -(C₁-C₁₂)-Alkyl,
   c) -(C₆-C₁₄)-Aryl, wobei Aryl unsubstituiert oder ein, zwei oder dreifach durch -(C₁-C₆)-Alkyl substituiert ist,
   d) -(C₃-C₈)-Cycloalkyl oder
   e) 4- bis 15-gliedriger Het-Ring steht,
3) -SO₃-R6,
4) -O-SO₂-R6,
5) -Si-R6 oder
6) Benzyl steht,
D für
1) -N(R2)-R3,
2) Halogen,
3) -O-SO₂-R2 oder
4) -O-C(O)-R2 steht,
   worin R2 und R3 gleich oder verschieden sind und unabhängig voneinander für
   a) Wasserstoffatom,
   b) -(C₁-C₁₂)-Alkyl,
   c) -(C₆-C₁₄)-Aryl, wobei Aryl unsubstituiert oder ein, zwei oder dreifach durch -(C₁-C₆)-Alkyl substituiert ist,
   d) -(C₃-C₈)-Cycloalkyl oder
   e) 4- bis 15-gliedriger Het-Ring, stehen.

Zu den Verbindungen der Formel II gehören insbesondere Verbindungen, worin
R1 für
   1) Wasserstoffatom,
   2) -(C₁-C₆)-Alkyl,
   3) Phenyl oder
   4) -(C₃-C₆)-Cycloalkyl, steht,
mit der Maßgabe, dass R1 nicht Wasserstoffatom ist, wenn P für Wasserstoffatom steht und D für -N(R2)-R3 steht, worin R2 für Wasserstoffatom steht und R3 für -(C₁-C₄)-Alkyl steht,
D für Chlor, Brom, Jod, Fluor oder -N(R2)-R3 steht, wobei
   R2 und R3 gleich oder verschieden sind und unabhängig voneinander für Wasserstoffatom oder -(C₁-C₄)-Alkyl, stehen,
P für
   1) Wasserstoffatom,
   2) -C(O)-O-R6, worin R6 für
      a) Wasserstoffatom,
      b) -(C₁-C₆)-Alkyl,
      c) -(C₆-C₁₄)-Aryl, wobei Aryl ausgewählt ist aus der Reihe Phenyl, Naphthyl, Anthryl oder Fluorenyl und worin Aryl unsubstituiert oder ein, zwei oder dreifach durch -(C₁-C₆)-Alkyl substituiert ist,
      d) -(C₃-C₆)-Cycloalkyl oder
      e) für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Benzimidazolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimida-zolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroiso-chinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl, steht,
   3) -SO₃-R6,
   4) -O-SO₂-R6,
   5) -Si-R6 oder
   6) Benzyl steht.

Ein Teil dieser Verbindungen sind Gegenstand der vorliegenden Erfindung gemäß Anspruch 1. Erfindungsgemäße neue Verbindungen der Formel II sind Verbindungen, worin
R1 für Wasserstoffatom oder Ethyl steht,
D für Chlor steht,
P für Wasserstoffatom, -O-Tosylat oder Benzyl steht.

Die Verbindungen der Formel II ergeben sich als Zwischenverbindungen der Formel VII in der Verfahrensvariante b) zur Herstellung der Verbindung der Formel I.

Beschrieben werden ferner Verbindungen der Formel IV, 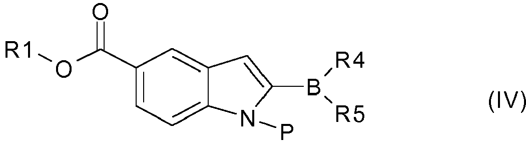 worin
R1 für
   1) Wasserstoffatom,
   2) -(C₁-C₁₂)-Alkyl,
   3) -(C₆-C₁₄)-Aryl,
   4) -(C₃-C₈)-Cycloalkyl oder
   5) 4- bis 15-gliedriger Het-Ring, steht,
R4 und R5 gleich oder verschieden sind und unabhängig voneinander für
   1) -OH,
   2) -O-(C₁-C₁₂)-Alkyl,
   3) -O-(C₆-C₁₄)-Aryl,
   4) -O-(C₃-C₈)-Cycloalkyl,
   5)-(C₁-C₁₂)-Alkyl oder
   6)-O-Het, wobei Het ein 4- bis 15-gliedriger Het-Ring bedeutet, stehen, oder
R4 und R5 bilden zusammen mit dem B-Atom an das sie gebunden sind einen Ring mit 4, 5, 6 oder 7 Kohlenstoffatomen im Ring und der Ring kann statt der jeweiligen Kohlenstoffatome zwei Sauerstoffatome oder zwei Sauerstoffatome und ein Stickstoffatom enthalten,
P für
   1) Wasserstoffatom,
   2) -C(O)-O-R6, worin R6 für
      a) Wasserstoffatom,
      b) -(C₁-C₁₂)-Alkyl,
      c) -(C₆-C₁₄)-Aryl, wobei Aryl unsubstituiert oder ein, zwei oder dreifach durch -(C₁-C₆)-Alkyl substituiert ist,
      d) -(C₃-C₈)-Cycloalkyl oder
      e) 4- bis 15-gliedriger Het-Ring steht,
   3) -SO₃-R6,
   4) -O-SO₂-R6,
   5) -Si-R6 oder
   6) Benzyl steht,
mit der Maßgabe, dass R4 und R5 nicht -OH oder -O-(C₁-C₁₂)-Alkyl sind, wenn P für -C(O)-O-(C₁-C₁₂)-Alkyl steht oder R1 für - (C₁-C₁₂)-Alkyl steht.
Zu diesen Verbindungen gehören z. B. Verbindungen der Formel IV, wobei
R1 für
   1) Wasserstoffatom,
   2) -(C₁-C₈)-Alkyl,
   3) -(C₆-C₁₄)-Aryl, wobei Aryl ausgewählt ist aus der Reihe Phenyl, Naphthyl, Anthryl oder Fluorenyl,
   4) -(C₃-C₆)-Cycloalkyl oder
   5) 4- bis 15-gliedriger Het-Ring worin Het für einen Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl oder Xanthenyl steht,
R4 und R5 gleich oder verschieden sind und unabhängig voneinander für
   1) -OH,
   2) -O-(C₁-C₁₂)-Alkyl,
   3) -O-(C₆-C₁₄)-Aryl, worin Aryl wie oben definiert ist,
   4) -O-(C₃-C₆)-Cycloalkyl,
   5) -(C₁-C₆)-Alkyl oder
   6) -O-Het, wobei Het ein 4- bis 15-gliedriger Het-Ring bedeutet und wie oben definiert ist, stehen, oder
R4 und R5 bilden zusammen mit dem B-Atom an das sie gebunden sind einen Ring aus der Reihe Borolan, Borinan, Borepan, Borocan, [1,3,2]Dioxaborolan, [1,3,2]Dioxaborinan, [1,3,2]Dioxaborepan, [1,3,2]Dioxaborocan oder [1,3,6,2]Dioxazaborocan, und
P für Wasserstoffatom steht.

Die Verbindungen der Formeln II, IVund XI eignen sich als Zwischenverbindungen zur Herstellung von IkB-Kinase Inhibitoren wie sie beispielsweise in WO 01/30774 A1 beschrieben werden.

Nachfolgend ist die Erfindung an Hand von Beispielen näher erläutert.
Endprodukte werden in der Regel durch ¹H-NMR (400 MHz, in DMSO-D6) bestimmt. Temperaturangaben in Grad Celsius, RT bedeutet Raumtemperatur (22 °C bis 26 °C). Verwendete Abkürzungen sind entweder erläutert oder entsprechen den üblichen Konventionen.

### Liste der Abkürzungen:

- Dba: Dibenzylidenaceton
- KHMDS: Kaliumhexamethyldisilazid
- KOtBu: Kaliumtertiär-butylat
- LDA: Lithiumdiisopropylamid
- LiHMDS: Lithiumhexamethyldisilazid
- LiTMP: Lithiumtetramethylpiperazid

| | |
|---|---|
| | 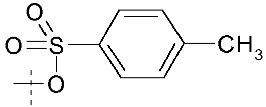 |
| OTos | entspricht -O-Tosylat |
| | 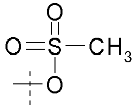 |
| OMes | entspricht -O-Mesylat |
| | 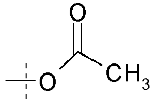 |
| OAc | entspricht -O-Acetat |
| Pd(PPh₃)₄ | Tetrakis(triphenylphosphin)palladium(O) |
| | 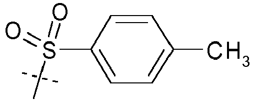 |
| Tos | entspricht Tosylat |
| TPPTS | Triphenylphosphintrisulfonat |
| THF | Tetrahydrofuran |

### Beispiel 1: Synthese von 2-(2-Chloropyrimidin-4-yl)-1H-indol-5-carbonsäureethylester

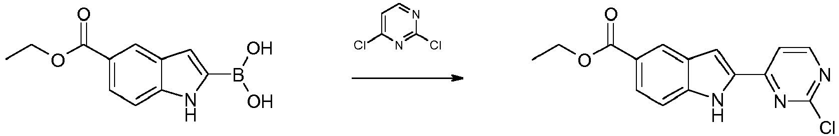

28 g (114 mmol) 2-Borono-5-ethoxycarbonylindol, 12 g (113 mmol) Natriumcarbonat und 17,2 g 2,4-(113 mmol) Dichloropyrimidin wurden in 412 ml Ethanol vorgelegt. Die klare Lösung wurde durch kräftiges Rühren und Durchleiten von Argon (20 Minuten) von Sauerstoff befreit. Bei RT wurden 2,67 g Terakis(triphenylphosphin)palladium(0) zugegeben. Das Gemisch wurde 2 Stunden (h) auf 65 °C bis 70 °C erhitzt. Anschließend wurden 112 ml Wasser und 112 ml 30 %ige Salzsäure zugeben und auf 0 °C abgekühlt. Nach Filtration und trocknen unter verminderten Druck wurden 37,3 g (93 % der Theorie) 2-(2-Chloropyrimidin-4-yl)-1H-indol-5-carbonsäureethylester erhalten (HPLC > 96%).

Die Bestimmung der Reinheit erfolgte durch Hochdruckflüssigchromatographie (HPLC):

| | | | | | |
|---|---|---|---|---|---|
| Säule: | Waters Symetry Shield RP8 3,9 * 150 | | | | |
| Temperatur: | 40 °C | | | | |
| Fluss: | 1 mL/ min | | | Inj ektionsvo lumen: | 10 µL |
| Druck: | 90 bar | | | UV: | 254 nm |
| Eluent: | A: Wasser/ Trifluoressigsäure (0,05%) | | | | |
| | B: Acetonitril/ Trifluoressigsäure (0,05%) | | | | |
| Zeit (min) | 0 | 15 | 20 | 25 | 30 |
| A (%) | 80 | 25 | 25 | 80 | 80 |
| B (%) | 20 | 75 | 75 | 20 | 20 |

Retentionszeit Titelverbindung: 12,6 min

### Beispiel 2: Synthese von 2-(2-Methylaminopyrimidin-4-yl)-1H-indol-5-carbonsäureethylester

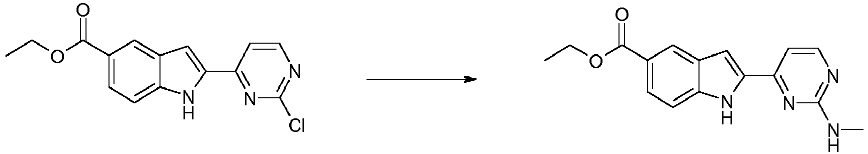

Es wurden 30 g (95,4 mmol) 2-(2-Chloropyrimidin-4-yl)-1H-indol-5-carbonsäureethylester vorgelegt und in 150 ml Ethanol suspendiert. Zu dieser Suspension wurden 53,9 g MethylaminLösung in Ethanol (8 M) gegeben und im Autoklaven 4 h auf 75 °C bis 80 °C erhitzt. Nach Einengen und Waschen mit Ethanol wurden 29,7 g 2-(2-Methylaminopyrimidin-4-yl)-1H-indol-5-carbonsäureethylester erhalten (97.6 HPLC-Fl.-%). LCMS: [M + H]⊕ 297,12 HPLC Methode wie in Beispiel 1; Retentionszeit Titelverbindung: 5,8 min

### Beispiel 3: Synthese von 2-(2-Methylaminopyrimidin-4-yl)-1H-indol-5-carbonsäure - Natriumsalz

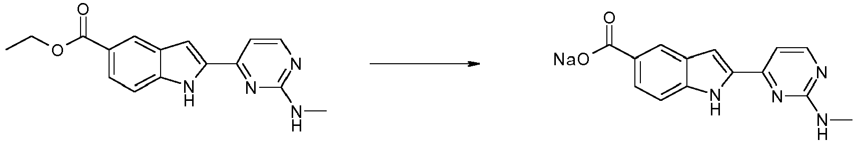

25 g 2-(2-Methylaminopyrimidin-4-yl)-1H-indol-5-carbonsäureethylester wurden mit 200 ml Ethanol und 24,5 g 33 %iger Natronlauge versetzt und 4 h auf 65°C bis 70 °C erhitzt. Nach dem Abkühlen wurde abgesaugt und der Niederschlag wurde mit 15 ml Ethanol/Wasser (9:1) gewaschen. Es wurden 24,5 g (87,6 % der Theorie) 2-(2-Methylaminopyrimidin-4-yl)-1H-indol-5-carbonsäure -Natriumsalz erhalten (98.1 HPLC-Flächen-%). LCMS: [M + H]⊕ 269,10
HPLC Methode wie in Beispiel 1 ; Retentionszeit Titelverbindung: 3,3 min

### Beispiel 4: Synthese von 2-Borono-5-ethoxycarbonylindol

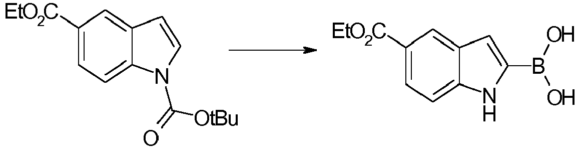

150 g (519 mmol) N-Boc-5-ethoxycarbonylindol und 192 ml (833 mmol) Triisopropylborat in 350 ml Toluol wurden bei 5 °C bis 10 °C mit 350 ml einer 1,8 molaren Lösung von LDA in THF versetzt. Es wurde 5 min nachgerührt und die Reaktionsmischung zu einer Lösung von 278 g
30 %iger Salzsäure und 940 ml Wasser gegeben. Anschließend wurde 30 min bei 5 °C bis 10 °C gerührt. Danach wurde filtriert und der Filterkuchen in 530 ml Ethanol suspendiert. Diese Suspension wurde bei 40 °C zu einer Lösung von 500 ml 30 %iger Salzsäure und 224 ml Ethanol gegeben. Anschließend wurde 2,5 h bei 40 °C bis 45 °C gerührt und bei 30 °C mit 380 ml Wasser versetzt. Dann wurde auf 10 °C bis 15 °C abgekühlt, 30 min bei dieser Temperatur nachgerührt und filtriert. Nach Trocknung unter vermindertem Druck wurden 79,5 g (61 % der Theorie) 2-Borono-5-ethoxycarbonylindol erhalten (HPLC: 92.7 Fl.-%).

## Patentansprüche

1. Verbindung der Formel II, 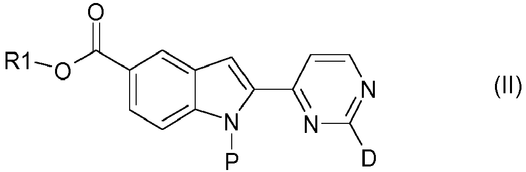 oder ein physiologisch verträgliches Salz der Verbindung der Formel II, wobei
R1 für Wasserstoffatom oder Ethyl steht,
D für Chlor steht,
P für Wasserstoffatom, -O-Tosylat oder Benzyl steht.

2. Verbindung der Formel II nach Anspruch 1, worin die Verbindung der Formel II die Struktur 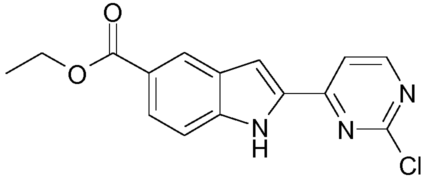 aufweist.

## Claims

1. Compound of the formula II 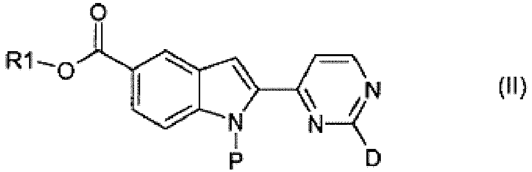 or a physiologically compatible salt of the compound of the formula II, where
R1 is a hydrogen atom or ethyl,
D is chlorine,
P is a hydrogen atom, -O-tosylate or benzyl.

2. Compound of the formula II according to Claim 1, in which the compound of the formula II has the structure

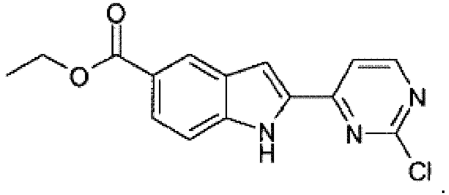

## Revendications

1. Composé de formule II 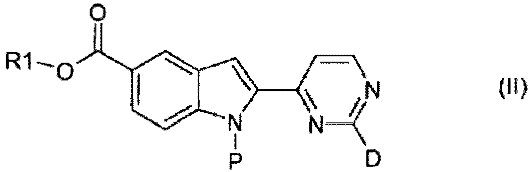 ou sel physiologiquement compatible du composé de formule II, dans laquelle
R1 représente un atome d'hydrogène ou éthyle,
D représente le chlore,
P représente un atome d'hydrogène, -O-tosylate ou benzyle.

2. Composé de formule II selon la revendication 1, dans lequel le composé de formule II présente la structure
